# EUROPÄISCHE PATENTSCHRIFT

(11) **EP 0 545 409 B1**
(45) Veröffentlichungstag und Bekanntmachung des Hinweises auf die Patenterteilung: **06.03.1996**
(21) Anmeldenummer: 92120645.4
(22) Anmeldetag: 03.12.1992
(51) Int. Cl.: C07F 7/08, C09K 19/40, C07J 51/00, G02F 1/13

(54) **Flüssigkristalle mit (Mehrfachsila)alkylflügelgruppen**
Liquid crystals with end groups containing more than one silane group
Cristaux liquides avec des groupes terminaux contenant plus d'un groupe silane

(30) Priorität: 06.12.1991 DE 4140352
(43) Veröffentlichungstag der Anmeldung: 09.06.1993
(73) Patentinhaber: Consortium für elektrochemische Industrie GmbH, D-81379 München (DE)
(72) Erfinder: Häberle, Norman, Dr., W-8000 München 21 (DE); Kreuzer, Franz-Heinrich, Dr., W-8033 Martinsried (DE); Krueger, Benno, Dr., W-8025 Unterhaching (DE); Zahn, Ingo, Dr., W-8000 München 40 (DE)

(56) Entgegenhaltungen:
- EP-A- 0 405 326

## Beschreibung

Die Erfindung betrifft neue Flüssigkristalle, die in den Flügelgruppen mehrere, untereinander mit Alkylenresten verbrückte Si-Atome enthalten, Verfahren zu deren Herstellung sowie deren Verwendung.

Die Verwendung von Silicium in Flüssigkristallen ist im Grundsatz bekannt, jedoch sind vor allem Polymer-Flüssigkristalle mit Poly- oder Oligosiloxan-Rückgrat in linearer oder auch cyclischer Anordnung mit unterschiedlichen mesogenen Seitenketten beschrieben, wie z. B. bei H. Finkelmann und G. Rehage in Macromol. Chem. Rapid Commun. 1, 31, (1980), oder auch in EP-A 29 162 (Finkelmann et al.; offengelegt am 27.05.1981 für Wacker-Chemie GmbH). Cyclosiloxane mit mesogenen Seitengruppen sind aus EP-A 60 335 (Kreuzer et al.; offengelegt am 22. 09.1982 für Consortium für elektrochemische Industrie GmbH) bekannt.

Außerdem sind auch monomere Flüssigkristalle bekannt, die in den Flügelgruppen entweder Trialkylsilylreste, wie in DE-A 38 27 600 (Hemmerling et al.; offengelegt am 15.02.1990 für Hoechst AG), verwenden oder Polymethyloligosiloxane, wie in JP 89/144491 (offengelegt am 06.06.1989, referiert in Chemical Abstracts, Vol. 111, 205 667 e (1989)), oder EP-A 404 140 (Haas et al.; offengelegt am 27.12.1990 für Consortium für elektrochemische Industrie GmbH). Durch diese Silanyl- bzw. Siloxanyl-Reste erhält man gegenüber den unsilylierten Verbindungen veränderte Eigenschaften. Dabei handelt es sich vor allem um Schmelzpunktsabsenkungen, Phasenverbreiterungen und Änderungen der Phasenarten. Die Trialkylsilylalkylhaltigen Flüssigkristalle rufen gegenüber unsilylierten Verbindungen jedoch nur kleinere Modifizierungen hervor. Die Siloxanyl-substituierten Derivate bewirken zwar in diesem Sinn mehr, sind jedoch chemisch wenig beständig und speziell bei größeren Siloxanen nur schwer in reiner Form herzustellen, da höhere Siloxane häufig nur in schwer zu trennenden Gemischen erhältlich sind. Durch die mangelnde chemische Stabilität der Si-O-Bindungen wird bedingt, daß die Einfügungen der Siloxanylreste die letzten chemischen Umsetzungen der Synthesen sein müssen, um nicht das gesamte Molekül wieder zu zerstören. Diese Forderung ist aber nicht erfüllbar, wenn, wie in Flüssigkristallen häufig vorkommend, Heteroatome wie Stickstoff oder Schwefel vorhanden sind, die eine Silylierung oder Siloxanierung verhindern.

Aufgabe der vorliegenden Erfindung war es, Flüssigkristalle bereitzustellen, die leichter herstellbar und chemisch beständiger sind und verbesserte flüssigkristalline Eigenschaften wie höhere Phasenbreiten und niedrigere Schmelzpunkte aufweisen.

Gegenstand der Erfindung sind Verbindungen der allgemeinen Formel I

M-(CH₂)ₙ-(O)ₘ-[-D-B-]_{q}-Y , (I)

in der bedeuten
- **M**: einen Rest aus 2 bis 5 Siliciumatomen in linearer, verzweigter oder cyclischer Anordnung, die untereinander mit Brückenelementen A verbunden sind, wobei die übrigen Valenzen der Siliciumatome mit Resten R abgesättigt sind;
- **A**: C₁- bis C₈-Alkylenreste oder Sauerstoff als Brückenelemente, mit der Maßgabe, daß je Rest M mindestens ein Rest der Bedeutung A einen C₁- bis C₈-Alkylenrest bedeutet;
- **R**: gleiche oder verschiedene, gegebenenfalls mit Fluor- oder Chloratomen oder Cyanogruppen substituierte geradkettige C₁- bis C₁₀-Alkyl- oder C₂- bis C₁₀-Alkenylreste, verzweigtkettige C₃- bis C₁₀-Alkyl- oder C₃- bis C₁₀-Alkenylreste, gegebenenfalls mit C₁- bis C₄-Alkyl-, C₁- bis C₄-Alkoxyresten, Fluor-, Chlor-, Bromatomen, Cyano-, Trifluormethyl- oder Nitroresten substituierte C₆- bis C₁₂-Cycloalkyl-, C₆- bis C₁₂-Cycloalkenyl-, C₆- bis C₁₂-Alkylcycloalkyl-, C₆- bis C₁₂-Alkylcycloalkenyl-, C₆- bis C₁₂-Aryl- oder C₆- bis C₁₂-Aralkylreste;
- **n**: eine ganze Zahl von 3 bis 12;
- **m**: 0 oder 1;
- **D**: gleiche oder verschiedene isocyclische oder heterocyclische gesättigte oder ungesättigte 5- oder 6-gliedrige Ringe;
- **B**: gleiche oder verschiedene Bindeglieder, die ausgewählt werden aus einer chemischen Bindung, einer Gruppe -COO-, -OOC-, -CH₂-CH₂-, -CH=CH-, -C≡C-, -CH=N-, -N=CH-, -CH₂-O-, -O-CH₂- und -N=N-;
- **q**: eine ganze Zahl von 1 bis 3;
- **Y**: ein Wasserstoffatom oder eine geradkettige oder verzweigtkettige C₁- bis C₁₀-Alkyl- oder C₁- bis C₁₀-Alkoxygruppe oder einen mit einem Wasserstoffatom versehenen oder substituierten Rest der Bedeutung D, wobei gegebenenfalls 1 bis 2 vorkommende Ring-Substituenten C₁- bis C₄-Alkyl-, C₁- bis C₄-Alkoxyreste, Fluor-, Chlor- oder Bromatome, Cyano-, Trifluormethyl- oder Nitroreste sein können, oder einen Cholesterylrest.

Soweit es von den Einzelstrukturen der Reste R her möglich ist, sind auch optische Isomere sowohl in reiner Form als auch in Form ihrer Gemische, beispielsweise der entsprechenden Racemate, Gegenstand der Erfindung.

Die Flügelgruppen M der erfindungsgemäßen Flüssigkristalle der allgemeinen Formel I weisen Silanreste auf. Diese Flügelgruppen sind über die Abstandgruppe (CH₂)ₙ-(O)ₘ mit einer stark variierbaren mesogenen Gruppe [-D-B-]_{q} verbunden.

Kleinere Werte für n als 3 bilden eine zu kurze Abstandgruppe und ergeben zusammen mit einem Sauerstoffatom, d.h. wenn m den Wert 1 hat, eine gegen chemische und thermische Einflüsse labile Abstandgruppe.

Bevorzugte Brückenelemente A sind geradkettige Alkylenreste, wie die Methylen-, 1,2-Ethylen, 1,3-Propylen-, 1,4-Butylen-, 1,5-Pentylen, 1,6-Hexylen-, 1,7-Heptylen oder 1,8-Octylengruppe.

Bevorzugte Reste D sind 1,4-Phenylenreste, 1,4-Cyclohexylidenreste, 1,4-Cyclohexenylidenreste, 2,5- oder 3,6-Pyridindiylreste, 2,5-Pyrimidindiylreste, 2,5-Pyridazindiylreste, 2,5-Triazindiylreste, 2,5-Dioxandiylreste, 2,5-Tetrahydrofurandiylreste, 1,3,4-Thiadiazol-2,5-diylreste oder 1,4-Bicyclo[2.2.2]-octandiylreste.

Beispiele für Reste R sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Alkenylreste, wie der Vinyl- und der Allylrest; Cycloalkylreste, wie Cyclopentyl-, Cyclohexyl-, Cycloheptylreste und Methylcyclohexylreste; Cycloalkenylreste, wie der Cyclohexenylrest; Arylreste, wie der Phenyl- und der Naphthylrest; Aralkylreste, wie o-, m-, p-Tolylreste; Xylylreste; Ethylphenylreste; Benzylreste; der alpha- und der β-Phenylethylrest.

Die vorstehenden Beispiele für Alkylreste beziehen sich auch auf die Alkylreste in den Alkoxygruppen.

Die Beispiele für die Alkyl- und Alkoxyreste beziehen sich auch auf Y.

Bevorzugte flüssigkristalline Verbindungen der allgemeinen Formel I sind Verbindungen, bei denen bedeuten:
- **M**: einen Rest aus 2 bis 5 Siliciumatomen in linearer oder verzweigter Anordnung, die untereinander mit Brückengliedern A verbunden sind, wobei die übrigen Valenzen der Siliciumatome mit Resten R abgesättigt sind;
- **A**: eine Methylen-, 1,2-Ethylen- oder 1,3-Propylengruppe oder ein Sauerstoffatom mit der Maßgabe, daß je Rest M mindestens ein Rest der Bedeutung A eine Methylen-, 1,2-Ethylen- oder eine 1,3-Propylengruppe bedeutet,
- **R**: gleiche oder verschiedene C₁- bis C₄-Alkylreste, wobei einer der am terminalen Siliciumatom befindliche Reste ausgewählt wird aus geradkettigen C₁-bis C₁₀-Alkyl- oder Alkenylresten, mit Fluor- oder Chloratomen substituierten C₁- bis C₅-Alkylresten, verzweigten C₃-bis C₁₀-Alkyl- oder C₃- bis C₁₀-Alkenylresten, gegebenenfalls mit C₁- bis C₄-Alkyl-, C₁- bis C₄-Alkoxyresten, Fluor-, Chlor-, Bromatomen, Cyano-, Trifluormethyl- oder Nitroresten substituierten C₆- bis C₁₂-Cycloalkyl-, C₆- bis C₁₂-Cycloalkenyl-, C₆- bis C₁₂-Alkylcycloalkyl-, C₆- bis C12-Alkylcycloalkenyl-, C₆-bis C₁₂-Aryl- oder C₆- bis C₁₂-Aralkylresten;
- **n**: eine ganze Zahl von 3 bis 10;
- **m**: den Wert 0 oder 1,
- **D**: gleiche oder verschiedene Reste, die ausgewählt werden aus 1,4-Phenylenresten, 1,4-Cyclohexylidenresten, 2,5- oder 3,6-Pyrimidindiylreste, 2,5-Dioxandiylreste oder 1,4-Bicyclo[2.2.2]-octandiylreste;
- **B**: gleiche oder verschiedene Bindeglieder, die ausgewählt werden aus einer chemischen Bindung, einer -COO-, -OOC-, -CH₂-CH₂- oder einer -CH=CH-Gruppe;
- **q**: eine ganze Zahl von 1 bis 3;
- **Y**: ein Wasserstoffatom oder eine geradkettige oder verzweigtkettige C₁- bis C₁₀-Alkyl- oder C₁- bis C₁₀-Alkoxygruppe oder einen mit einem Wasserstoffatom versehenen oder substituierten Phenylrest oder mit einem Wasserstoffatom versehenen oder substituierten Cyclohexyl- oder Cyclohexenylrest, wobei gegebenenfalls 1 oder 2 vorkommende Substituenten C₁- bis C₄-Alkyl-, C₁- bis C₄-Alkoxyreste, Fluor- oder Chloratome oder Cyanoreste sein können oder einen Chloresterylrest.

Bei den besonders bevorzugten Verbindungen der allgemeinen Formel I bedeuten:
- **n**: eine ganze Zahl von 4 bis 8;
- **m**: den Wert 0 oder 1;
- **D**: gleiche oder verschiedene Reste, die ausgewählt werden aus 1,4-Phenylen- oder 1,4-Cyclohexylidenresten;
- **B**: gleiche oder verschiedene Bindeglieder, die ausgewählt werden aus einer chemischen Bindung, einer -COO-, -OOC- oder einer -CH₂-CH₂-Gruppe;
- **q**: den Wert 1 oder 2;
- **Y**: ein Wasserstoffatom oder eine geradkettige oder verzweigtkettige C₁- bis C₁₀-Alkylgruppe oder C₁- bis C₁₀-Alkoxygruppe oder einen mit einem Wasserstoffatom versehenen oder substituierten Phenyl- oder Cyclohexylrest, wobei gegebenenfalls 1 oder 2 vorkommende Substituenten C₁- bis C₄-Alkyl-, C₁- bis C₄-Alkoxyreste, Fluor- oder Chloratome oder Cyanoreste sein können oder einen Cholesterylrest;
- **M**: einen Rest der allgemeinen Formel II:

R-[Si(CH₃)₂-A]ₚ-SiR₂-, (II)
in der bedeuten
- **A**: eine Methylengruppe;
- **R**: eine geradkettige C₁- bis C₄-Alkylgruppe oder einen Phenylrest;
- **p**: den Wert 1 oder 2.

Vorzugsweise sind in der allgemeinen Formel II alle Reste R Methylgruppen.

Die erfindungsgemäßen Verbindungen werden erfindungsgemäß in an sich bekannter Weise durch folgende Verfahren hergestellt:

### Verfahren a:

Die Verbindungen der vorstehenden allgemeinen Formel I können durch Umsetzung von Verbindungen der allgemeinen Formel MH mit Verbindungen der allgemeinen Formel III

H₂=CH-(CH₂)ₙ₋₂-(O)ₘ-[-D-B-]_{q}-Y (III)

in Gegenwart von mindestens einem Metall der Platingruppe und/oder dessen Verbindungen hergestellt werden, wobei M, A, R, n, m, D, B, q, Y, und p die in den vorstehenden allgemeinen Formeln I und II angegebenen Bedeutungen aufweisen, mit der Maßgabe, daß das Wasserstoffatom in MH an ein Siliciumatom gebunden ist.

Die benötigten Silane der allgemeinen Formel MH, in der M und damit R, A und p die für die allgemeine Formel I angegebenen Bedeutungen annehmen, sind nach bekannten Verfahren wie z. B. Grignard-Synthesen von Alkylsilylmethylmagnesiumhalogeniden mit Alkylsilylhalogen-Verbindungen oder von Hydrosilylierungsreaktionen von Alkyl-Si-H-Derivaten mit Vinyl-(allgemein: Alkenyl)alkylsilan-Derivaten unter Pt-Katalyse oder aber durch Pyrolyse von Alkylsilanen erhältlich. In gleicher Weise sind auch verzweigte Silane erhältlich, die aber auch durch Pyrolyse von Alkylsilanen herstellbar sind. Nach dem letzteren Verfahren sind auch Oligosilacycloalkane erhältlich. Als Beispiel sei die Herstellung von 1,1,3,3,5-Pentamethyl-1,3,5-trisilacyclohexan (G. Fritz et al., Z. anorg. all. Chem. 1980, 460, S. 115 - 143) genannt.

Die Herstellung von Verbindungen mit endständigen und damit hydrosilylierbaren Doppelbindungen der allgemeinen Formel III, in der n, m, D, B, q und Y die für die allgemeine Formel I genannten Bedeutungen annehmen, ist ebenfalls bekannt.

Beispielsweise kann der Alkenylrest eingeführt werden durch Umsetzung der entsprechenden Alkenylhalogenide mit entsprechenden metallorganischen Benzolderivaten, insbesondere den Grignardverbindungen oder Organolithiumverbindungen. Bevorzugte Beispiele für solche Benzolderivate sind 4-Halogenphenylmagnesiumhalogenide. Die Umsetzung von Alkenylhalogenid mit dem Benzolderivat erfolgt vorzugsweise in einem inerten Lösungsmittel(gemisch), beispielsweise Diethylether, Methyl-tert.-butylether, Tetrahydrofuran, 1,4-Dioxan, Kohlenwasserstoffen und deren Gemischen, wie Benzol, Toluol, Xylole, Hexanisomerengemischen oder Petrolether. Die Umsetzung wird vorzugsweise bei Temperaturen von -100°C bis +100°C, insbesondere bei Drücken von 0,09 bis 0,11 MPa (abs.) durchgeführt. Die Reaktion kann unter Umständen durch Ultraschall beschleunigt werden.

So hergestellte 4-(ω-Alkenyl)-1-halogenbenzole können in an sich bekannter Weise u. a. ein zweites Mal mit Magnesium zu den entsprechenden Organomagnesiumhalogeniden und anschließend mit CO₂ zu 4-(ω-Alkenyl)benzoesäuren und schließlich zu den entsprechenden, gegebenenfalls substituierten Phenylestern umgesetzt werden.

4-(ω-Alkenyl)phenolderivate können erhalten werden durch Umsetzung von 4-ω-Alkenylmagnesiumhalogeniden mit an der Hydroxylgruppe geschützten 4-Halogenphenolen, gegebenenfalls unter Katalyse von Dilithiumtetrachlorocuprat. Die so erhältlichen Phenole können mit entsprechenden Säuren oder Säurederivaten verestert werden. Für die grundsätzlich bekannte Herstellung von 4-(ω-Alkenyl)phenylderivaten wird auf DE-A-39 35 638 sowie DE-A-40 22 151 verwiesen.

Durch die Umsetzung von 4-(ω-Alkenyl)phenylmagnesiumhalogeniden (herstellbar wie oben erläutert) mit N,N-Dimethylformamid sind 4-(ω-Alkenyl)benzaldehyde zugänglich, welche wiederum mit primären Aminen - beispielsweise mit den käuflich erhältlichen 4-Alkylanilinen - zu den entsprechenden Azomethinen (Schiff'schen Basen) reagieren.

Setzt man die nach obenstehendem Verfahren herstellbaren 4-(ω-Alkenyl)benzaldehyde mit geeigneten Derivaten, beispielsweise Alkanphosphonsäureester (Wittig-Horner-Reaktion), um, so erhält man 4-(ω-Alkenyl)phenylethylendiylderivate.

Gemäß EP-A-168 683, Beispiel 26, kann man 1-(ω-Alkenyl)-4-nitrobenzol zu 4,4'-Di-(ω-alkenyl)azoxybenzolen reduzieren, beispielsweise mittels Magnesiumspänen in Methanol als Lösungsmittel. Dieses wiederum läßt sich in bekannter Weise zum entsprechenden Azobenzol reduzieren, beispielsweise durch Zink und Natronlauge.

Heterocyclische Elemente D sind ebenfalls in bekannter Weise zugänglich. So können anstelle von aromatischen Ringen auch - zum Teil auch käufliche - substituierte Heterocyclen eingesetzt werden oder aber ringsynthetisch hergestellt werden. So können die Dioxane beispielsweise durch Umsetzung von 2-(M-Alkyl)1,3-diolen mit 4-substituierten Benzaldehydderivaten erhalten werden. Die dazu notwendigen 2-(M-Alkyl)-1,3-diole sind durch Reduktion von 2-(M-Alkyl)malonsäurediestern mit Lithiumaluminiumhydrid erhältlich, diese Diester durch Hydrosilylierung von zum Teil auch käuflichen 2-(ω-Alkenyl)malonsäurediestern. Beispiele für diese Reaktionsfolge sind im Beispielteil enthalten.

Die Pyrimidin-Derivate sind in an sich bekannter Weise z. B. durch Umsetzung von 4-(M-Alkyloxy)benzonitrilen über 4-(M-Alkyloxy)benzimidchloride zu 4-(M-Alkyloxy)benzamidinen zu erhalten. Diese Benzamidin-Derivate können in ebenfalls bekannter Weise mit substituierten Malondialdehyd- bis(dialkyl)acetalen zu den gesuchten Pyrimidin-derivaten cyclisiert werden.

Die katalysierte Hydrosilylierung der endständigen Doppelbindungen ist ebenfalls eine bekannte, vielfach benutzte Methode. Beispiele für katalytisch wirksame Metalle der Platingruppe und/oder deren Verbindungen - nachfolgend Platinkatalysator genannt - , die das erfindungsgemäße Verfahren beschleunigen können, sind Platin, Palladium, Rhodium, Iridium und deren Verbindungen, vorzugsweise Platin und/oder dessen Verbindungen. Es können hier alle Katalysatoren eingesetzt werden, die auch bisher zur Addition von direkt an Si-Atome gebundenen Wasserstoffatomen an ungesättigte aliphatische Verbindungen eingesetzt wurden. Beispiele für solche Katalysatoren sind metallisches und feinverteiltes Platin, das sich auf Trägern, wie Siliciumdioxyd, Aluminiumoxyd oder Aktivkohle befinden kann, Verbindungen oder Komplexe von Platin, wie Platinhalogenide, z. B. PtCl₄, H₂PtCl₆ . 6 H₂O, Na₂PtCl₄ . 4 H₂O, Platin-Olefin-Komplexe, Platin-Alkohol-Komplexe, Platin-Alkoholat-Komplexe, Platin-Ether-Komplexe, Platin-Aldehyd-Komplexe, Platin-Keton-Komplexe, einschließlich Umsetzungsprodukten aus H₂PtCl₆ . 6 H₂O und Cyclohexanon, Platin-Vinylsiloxankomplexe mit oder ohne Gehalt an nachweisbarem anorganisch gebundenem Halogen, Bis-(gamma-picolin)-platindichlorid, Trimethylendipyridinplatindichlorid, Dicyclopentadienylplatindichlorid, Dimethylsulfoxydethylenplatin-(II)-dichlorid sowie Umsetzungsprodukte von Platintetrachlorid mit Olefin und primärem Amin oder sekundärem Amin oder primärem und sekundärem Amin, wie das Umsetzungsprodukt aus in 1-Octen gelöstem Platintetrachlorid mit sec.-Butylamin, oder Ammonium-Platinkomplexe gemäß EP 110 370.

Platinkatalysator wird vorzugsweise zugegeben in Mengen von 0,02 bis 50 Molprozent, jeweils berechnet als elementares Platin und bezogen auf die Molzahl derjenigen Reaktionskomponente, welche im Unterschuß oder in stöchiometrischer Menge vorliegt.

Die Reaktion wird vorzugsweise bei Temperaturen von 0°C bis 150°C, insbesondere 10 bis 100°C, vorzugsweise bei Drücken von 0,05 MPa bis 2,0 MPa durchgeführt.

Sollte das Silan MH oder die Verbindung der allgemeinen Formel III sehr reaktionsträge sein, kann auch bei höheren Temperaturen, höheren Drücken und in Anwesenheit von mehr Platinkatalysator gearbeitet werden.

Vorzugsweise wird die Reaktion in einem inerten Lösungsmittel durchgeführt, welches insbesondere aprotisch sein sollte; Lösungsmittel oder Lösungsmittelgemische mit einem Siedepunkt bzw. Siedebereich von bis zu 160°C, insbesondere von bis zu 120°C, bei jeweils 0,1 MPa (abs.) sind bevorzugt. Beispiele für Lösungsmittel sind Ester, wie Methylacetat, Ethylacetat, n- und iso-Propylacetat, n-, sec.- und t.-Butylacetat, Ethylformiat und Diethylcarbonat; Ether, wie Dioxan, Tetrahydrofuran, Diethylether, Di-n-propylether, Diisopropylether, Di-n-butylether, Ethylenglycoldimethylether, Ethylenglycolmonoethylether, Diethylenglycoldimethylether und Anisol; chlorierte Kohlenwasserstoffe, wie Dichlormethan, Trichlormethan, Tetrachlormethan, 1,2-Dichlorethan, Trichlorethylen, Tetrachlorethylen und Chlorbenzol; Kohlenwasserstoffe, wie Pentan, n-Hexan, Hexan-Isomerengemische, Cyclohexan, Heptan, Octan, Waschbenzin, Petrolether, Benzol, Toluol, Xylole; Ketone, wie Aceton, Methylethylketon, Methylisobutylketon oder Gemische dieser Lösungsmittel.

Die Bezeichnung Lösungsmittel bedeutet nicht, daß sich alle Reaktionskomponenten in diesem lösen müssen. Die Reaktion kann auch in einer Suspension oder Emulsion eines oder mehrerer Reaktionspartner durchgeführt werden. Die Reaktion kann auch in einem Lösungsmittelgemisch mit einer Mischungslücke ausgeführt werden, wobei in jeder der Mischphasen jeweils mindestens ein Reaktionspartner löslich ist.

Vorzugsweise wird die Silylkomponente MH im erfindungsgemäßen Verfahren mit der zu addierenden Verbindung nach der obenstehenden Reaktionsgleichung im Molverhältnis 1:2 bis 2:1, insbesondere von 1:1,1 bis 1,1:1, eingesetzt.

Nähere Einzelheiten zu den bekannten Herstellverfahren sind aus Houben-Weyl, Methoden der organischen Chemie, Georg Thieme Verlag, Stuttgart und New York, zu entnehmen. Weitere Möglichkeiten sind im Beispielteil aufgeführt.

### Verfahren b:

Die Verbindungen der allgemeinen Formel I können durch Umsetzung von Halogensilanverbindungen der allgemeinen Formel M-Hal, in der Hal ein Chlor-, Brom- oder Jodatom bedeutet, mit metallorganischen Verbindungen der allgemeinen Formel IV

T-(CH₂)ₙ-(O)ₘ-[-D-B]_{q}-Y , (IV)

in der T ein Alkalimetallatom oder Mg-Hal bedeutet, wobei Hal die vorstehenden Bedeutungen aufweist, unter Abspaltung eines Metallhalogenids T-Hal hergestellt werden. M, A, R, n, m, D, B, q, Y, und p weisen im Verfahren b die in den vorstehenden allgemeinen Formeln I und II angegebenen Bedeutungen auf, mit der Maßgabe, daß für den Fall, daß B und/oder Y mit Alkalimetallen oder Magnesium oder Verbindungen der allgemeinen Formel IV reagieren, B und/oder Y erst nach erfolgter Umsetzung von M-Hal mit Verbindungen der allgemeinen Formel IV eingeführt werden.

Gruppen B und Y, die mit Alkalimetallen oder Magnesium oder Verbindungen der allgemeinen Formel IV reagieren, sind beispielsweise Carboxylderivate und Cyanogruppen.

Bei der Reaktion nach Verfahren b können Katalysatoren wie Eisen(-III)chlorid oder Dilithiumtetrachlorocuprat und/oder Dilithiumtetrabromcuprat oder Dichlor(bis-diphenylphosphinopropan)nickel beschleunigend wirken. Bezüglich der Reihenfolge der Reaktionsschritte, die zu verschiedenen Elementen D, B, q und y führen, gilt das für Verfahren a) Gesagte entsprechend.

### Verfahren c:

Die Verbindungen der allgemeinen Formel I können durch Umsetzung von Verbindungen der allgemeinen Formel V

M-(CH₂)ₙ-(O)ₘ-[-D-B-]ᵣ₋₁-D-G (V)

mit Verbindungen der allgemeinen Formel VI

Q-[-D-B-]ₛ-Y (VI)

hergestellt werden. Dabei weisen r und s jeweils ganze Zahlen von 0 bis 3 , deren Summe q beträgt, G und Q jeweils einen der Reste -OH, -OLi, -ONa, -OK, -O-C₁- bis -O-C₄-Alkyl, -COOH, -COBr, -COCl, -NH₂, -O-tos oder -MgHal und M, A, R, n, m, D, B, q, Y, und p die in den vorstehenden allgemeinen Formeln I und II angegebenen und Hal die Bedeutungen Chlor-, Brom- oder Iodatom auf. Bei dem Verfahren c werden B-Gruppen unter Abspaltung von Verbindungen W mit der Bedeutung Wasser, C₁-bis C₄-Alkanol, HHal, MgHal₂, LiHal, NaHal, KHal, Li-O-tos, Na-O-tos oder K-O-tos gebildet. Die (4-Methylphenylsulfonyl)-Gruppe ist mit tos bezeichnet.

Die zur Herstellung der Verbindungen der allgemeinen Formeln V und VI benötigten Ausgangsmaterialien sind nach den bei Verfahren a) beschriebenen Wegen zugänglich.

### Weitere Verfahren

Eine weitere Möglichkeit zur Herstellung der Verbindungen der allgemeinen Formel I ist die Umsetzung von Verbindungen der allgemeinen Formel VII

M-(CH₂)ₙ-Z (VII)

mit Verbindungen der allgemeinen Formel VIII

N-O-[-D-B-]_{q}-Y (VIII)

Z bedeutet dabei ein Chlor-, Brom- oder Iodatom oder die Gruppe O-tos. N bedeutet ein Wasserstoff- oder Natriumatom. Bei der Umsetzung werden Chlor-, Brom- oder Iodwasserstoff oder Na-O-tos abgespalten.

Die erfindungsgemäßen bzw. erfindungsgemäß herstellbaren Flüssigkristalle mit (Mehrfachsila)alkyl-Flügelgruppen können beispielsweise in Anzeigevorrichtungen verwendet werden, besonders in Anzeigevorrichtungen, welche unter Verwendung von smektischen Flüssigkristallen oder deren Mischungen hergestellt werden. Dabei können sowohl die reinen erfindungsgemäßen Verbindungen, als auch deren Mischungen untereinander sowie insbesondere auch Mischungen mit Flüssigkristallverbindungen anderer Bauart verwendet werden. Die erfindungsgemäßen Verbindungen eignen sich zur Herstellung von nematischen, cholesterischen und smektischen Gemischen, vor allem für Gemische, welche eine smektische C-Phase ausbilden können. Sie können aber auch als Zusätze zu nematischen, smektischen oder cholesterischen Phasen Verwendung finden. Mit Hilfe der erfindungsgemäßen Flüssigkristalle mit (Mehrfachsila)alkyl-Flügelgruppen lassen sich sowohl flüssigkristalline Basismischungen herstellen als auch die Eigenschaften bereits fertiggestellter Basismischungen, wie beispielsweise die optische Anisotropie, die elektrische Anisotropie, die spontane Polarisation, die Viskosität, der Tiltwinkel, der Pitch und das Phasenverhalten günstig verändern.

Durch die (Mehrfachsila)alkyl-Flügelgruppen werden bei den erfindungsgemäßen Flüssigkristallen gegenüber den Flüssigkristallen mit Siloxanylalkyl-Flügelgruppen nach DE-A-30 20 509 wesentlich günstigere Eigenschaften erhalten. Selbst bei sonst völlig gleichem Molekülbau sind bei den erfindungsgemäßen Verbindungen die Flüssigkristall-Phasen breiter, weisen niedrigere Schmelzpunkte und Klärpunkte auf als (Siloxanylalkyl)-flügelgruppenhaltige Flüssigkristalle und sind wegen der Silicium-Kohlenstoff-Bindungen gegen chemische Umsetzungen wesentlich stabiler.

Trotz der bekannten Empfindlichkeit flüssigkristalliner Eigenschaften gegenüber Änderungen im Molekülbau einerseits und gegenüber großen Substituenten im Flügelgruppenanteil andererseits sind große Variationsbreiten im Bau der Flügelgruppen zu verzeichnen. Trotz der sehr verschiedenartigen Substituenten an den Si-Atomen der Flügelgruppen bleiben die smektischen C-Phasen weitgehend erhalten, während beispielsweise die als Ausgangsverbindungen benützten (ω-Alkenyl)flügelgruppen-Verbindungen oft nur nematische Phasen aufweisen oder kristallin-monotrope Verbindungen sind. Die große Variationsbreite im Flügelgruppenanteil der erfindungsgemäßen Verbindungen gestattet auch die Anpassung der Moleküle an sonstige gewünschte Eigenschaften der herzustellenden Flüssigkristalle und macht andererseits die Restanteile der Moleküle (Mesogenanteile) auf diese Weise in großem Umfang frei wählbar.

Der Anteil der erfindungsgemäßen Flüssigkristalle mit (Mehrfachsila)alkyl-Flügelgruppen in Flüssigkristallmischungen kann je nach Verwendungszweck in breiten Grenzen variieren. Er kann beispielsweise von 1 Gewichtsprozent bis zu 100 Gewichtsprozent betragen.

In den nachfolgenden Tabellen und Beispielen sind, falls jeweils nicht anders angegeben,
a) alle Mengenangaben auf das Gewicht bezogen;
b) alle Drücke 0,10 MPa (abs.);
c) alle Temperaturen 20°C.

Die Phasenbeschreibungen sind wie folgt abgekürzt dargestellt:
d) Die Zahlenwerte bedeuten Übergangstemperaturen, gemessen in °C;
e) Die Phasentypen sind wie folgt charakterisiert:
   - I :: isotrope Phase,
   - N :: nematische Phase,
   - N* :: cholesterische Phase,
   - S_{A} :: smektische A-Phase,
   - S_{C} :: smektische C-Phase, usw. für andere smektische Phasen laut Index-Buchstabe
   - S_{C}* :: chirale smektische C-Phase,
   - S :: smektischer Zustand, dessen Typ nicht bestimmt wurde,
   - C :: kristallin,
   - G :: Glaszustand.
f) Phasenbeschreibungen in Klammern zeigen unterkühlbare Phasen an.

### Beispiel 1

### a) Herstellung von 4-(ω-Alkenyl)-1-chlorbenzol-Derivaten

Eine Lösung von 294 g (2 Mol) 1,4-Dichlorbenzol in 500 ml wasserfreiem Tetrahydrofuran wurde unter Stickstoff bei 80°C zu einer gerührten Suspension von 48,6 g (2,0 Mol) Magnesiumspänen innerhalb von 110 min. zugetropft. Anschließend wurde noch 2 Stunden auf 80 - 84°C erhitzt, dann vom überschüssigen Magnesium dekantiert und diese Lösung der Grignardverbindung bei 80°C innerhalb von 30 min. zu einer gerührten Lösung von 194 g (1,8 Mol) 6-Brom-1-hexen (käuflich bei Fluka GmbH, D - 7910 Neu-Ulm) in 200 ml Tetrahydrofuran zugetropft. Danach wurde noch 4 Stunden bei 80°C gerührt und das Gemisch auf Eis gegossen. Nach Ansäuern mit Salzsäure wurde dreimal mit einem 1 : 1-Gemisch Diethylether-/Methyltert.butylether extrahiert, die Etherfraktionen mit Aktivkohle durchgerührt und über Natriumsulfat getrocknet. Nach Einengen des Filtrats wurde der Rückstand bei vermindertem Druck fraktioniert. Bei einem Druck von 13 hPa und einer Temperatur von 116 - 118°C wurden 156 g (entsprechend 40 % Ausbeute der Theorie) an 4-(5-Hexenyl)-1-chlorbenzol erhalten. Analog wurden hergestellt:
4-Allyl-1-chlorbenzol; (Kp. 66°C bei 13 hPa)
4-(3-Butenyl)-1-chlorbenzol; (Kp. 87 - 89°C bei 13 hPa)
4-(4-Pentenyl)-1-chlorbenzol; (Kp. 102 - 105°C bei 13 hPa)
4-(8-Nonenyl)-1-chlorbenzol; (Kp. 87°C bei 0,3 hPa)
4-(11-Dodecenyl)-1-chlorbenzol; (Kp. 147 - 150°C bei 0,2 hPa)

### b) Herstellung von 4-(ω-Alkenyl)benzoesäuren

Aus 129 g (0,77 Mol) 4-(3-Butenyl)-1-chlorbenzol und 30 g (1,23 Mol) Magnesium wurde bei 70°C in üblicher Weise eine Grignardlösung hergestellt, abgekühlt, vom überschüssigen Magnesium befreit und diese Lösung bei 15 - 25°C in 300 ml CO₂-gesättigtes Tetrahydrofuran eingetropft, wobei weiterhin CO₂ eingeleitet wurde. Nach beendeter Zugabe wurde 1,5 Std. bei Raumtemperatur nachgerührt und dann das Gemisch auf 200 g Eis gegossen. Danach wurde angesäuert, 300 ml Diethylether zugegeben, ausgeschüttelt und die Phasen getrennt. Nach Waschen der Etherphase mit Wasser (2 x) und Trocknen wurde eingeengt. Der Rückstand wurde aus 600 ml Hexan umkristallisiert. Erhalten wurden 102,2 g 4-(3-Butenyl)-benzoesäure, entsprechend 77,4 % Ausbeute der Theorie. Die Substanz zeigte folgendes Phasenverhalten: C 117 N 130 I.

Analog wurde hergestellt:
4-Allylbenzoesäure C 98 N 122 I
4-(4-Pentenyl)benzoesäure C 76 N 96 I
4-(5-Hexenyl)benzoesäure C 84 N 117 I
4-(9-Decenyl)benzoesäure C 80 S_{C} 85 N 108 I
4-(11-Dodecenyl)benzoesäure C 83 S_{C} 107 I

Aus diesen Carbonsäuren wurden in an sich bekannter Weise durch Umsetzung mit Thionylchlorid die entsprechenden Carbonsäurechloride hergestellt.
4-Allylbenzoesäurechlorid Kp. 115 - 116°C bei 8,5 hPa
4-(3-Butenyl)benzoesäurechlorid Kp. 130 - 132°C bei 13 hPa
4-(4-Pentenyl)benzoesäurechlorid Kp. 101°C bei 1,5 hPa
4-(5-Hexenyl)benzoesäurechlorid Kp. 95°C bei 0,1 hPa
4-(9-Decenyl)benzoesäurechlorid Kp. 160 - 162°C bei 0,04 hPa

### c) Herstellung von 4-(ω-Alkenyl)benzoesäuren-(subst.)phenylesterderivaten

Die Veresterung geschieht in bekannter Weise durch Umsetzung eines der Säurechloride nach b) mit einem geeigneten Phenolderivat unter Abziehen des gebildeten Chlorwasserstoffs oder eventuell unter Verwendung einer Hilfsbase wie Pyridin oder Triethylamin in einem inerten Lösungsmittel wie Toluol oder Xylol. Die benötigten Phenolderivate sind teils käuflich oder sind in an sich bekannter Weise darstellbar.

### Herstellung von 4-(4-Propylcyclohex-1-enyl)phenol

Käufliches 4-Bromphenol wurde nach L. Santucci und H. Gilman, J. Am. Chem. Soc. 1958, 80, S. 4537 mit käuflichem Dihydropyran in den Tetrahydropyranylether (THP-Ether) des 4-Bromphenols umgewandelt (Ausbeute 90 % d. Th., Kp. bei 0,2 hPa 105°C). 2 ml dieses THP-Ethers wurden unter Schutzgas zu 7,3 g Magnesiumspänen, die mit Tetrahydrofuran befeuchtet waren, zugegeben und auf 50°C erwärmt, bis die GrignardReaktion in Gang gekommen war. Anschließend wurde unter Rühren bei 50 - 60°C der Rest von insgesamt 64,3 g (0,25 Mol) des THP-Ethers, gelöst in THF, innerhalb 2 Stunden zugetropft. Nach dem Erkalten wurde vom überschüssigen Magnesium dekantiert und die Lösung bei 10°C in 2,5 Std. zu einer Lösung von 34,8 g (0,25 Mol) käuflichen 4-Propylcyclohexanons (EMS, CH-5605 Dottikon) in 300 ml Toluol zugetropft. Nach einer Stunde Nachreaktion bei 80°C wurde abgekühlt, auf Eis gegossen, angesäuert und die entstehenden Phasen getrennt aufgearbeitet. Das Rohprodukt wurde mit Methanol/Salzsäure 1 Stunde bei 65°C gespalten, dann eingeengt, in Xylol gelöst, mit 1 g p-Toluolsulfonsäure versetzt und eine Stunde am Wasserabscheider auf Rückfluß erhitzt. Danach wurde eingeengt und der Rückstand im Hochvakuum fraktioniert. Eine Fraktion, die bei 0,025 hPa und 160 - 190°C destillierte, wurde anschließend aus Cyclohexan umkristallisiert. Erhalten wurden 11 g (20,4 % der Theorie) des gesuchten Produkts, das bei 82°C schmilzt.

In gleicher Weise wurde aus dem THP-Ether des 4'-Brombiphenylols mit 23,3 % der theoretischen Ausbeute 4-(4-Propylcyclohex-1-en-1-yl-)phenyl-phenol (C₂₈H₃₂O₂; MG 400,6) mit einem Schmelzpunkt von 221°C hergestellt.

### Herstellung von 4-[(4-Chlorphenyl)ethyl]phenol

Von 25,7 g (0,1 Mol) des oben genannten 4-Bromphenyl-THP-Ethers wurde wie im vorigen Abschnitt eine Grignardlösung hergestellt und bei 70°C zu einem Gemisch aus 31,1 g Toluolsulfonsäure-(4-chlorphenyl)ethylester (aus käuflichem 4-Chlorphenylethanol (Aldrich - D - 7924 Steinheim) mit üblicher Tosylierung herstellbar; Fp. 80°C), einer katalytischen Menge Dilithiumtetrachlorocuprat und 50 ml Toluol zugetropft. Anschließend wurde noch 2 Stunden bei 90°C nacherhitzt und dann abgekühlt. Schon im Lauf der Reaktion entstand ein voluminöser Niederschlag von Lithiumtosylat. Der gesamte Ansatz wurde auf Eis gegossen, angesäuert und mit Wasser verdünnt, bis zwei trennbare, klare Phasen entstanden. Nach Phasentrennung und Aufarbeitung der organischen Phase wurde mit Methanol/Salzsäure das Phenol-Derivat freigesetzt, neutralisiert und eingeengt. Durch Fraktionierung des Rückstands wurden bei 0,07 hPa und 155 - 170°C 76 g Rohprodukt erhalten, das aus Toluol/Petrolether umkristallisiert wurde und dann einen Schmelzpunkt von 108 109°C aufwies.

Durch die beschriebene Veresterung wurden die nachstehenden ω-ungesättigten Ester hergestellt:
4-(9-Decenyl)benzoesäure-(4-[4-propylcyclohex-1-en-1-yl)biphenylyl]ester; C₃₈H₄₆O₂
   Phasenverhalten S_{H} 73 S_{G} 144 S_{B} 174 S_{C} 214 S_{A} 236 N 279 I;
4-[4-(3-Butenyl)phenylethyl]benzoesäure-(4-cyanobiphenylyl)ester;
   Phasenverhalten: C 123 N 293 I; C₃₂H₂₇NO₂
4-(4-Pentenyl)benzoesäure-[4-(2-(4-methoxyphenyl)ethyl)]phenylester;
   Phasenverhalten: C 74 N 118 I; C₂₇H₂₈O₃
4-(3-Butenyl)benzoesäure-(4-propylcyclohex-1-en-1-yl)phenylester;
   Phasenverhalten: C 81 N 181 I; C₂₆H₃₀O₂
4-(8-Nonenyloxy)benzoesäure-[4-(4-propylcyclohex-1-en-1-yl)biphenylyl]ester;
   Phasenverhalten: S_{H} 107 S_{G} 114 S_{I} 135 S_{C} 208 N 299 I; C₃₇H₄₄O₃
4-(3-Butenyl)benzoesäure-[4-(4-nonylcyclohexyl)phenyl]ester
   Phasenverhalten: C 78 S_{B} 112 N 168 I; C₃₂H₄₄O₂

### d) Herstellung von 4-(ω-oligoalkyl/arylsilyl)alkylbenzoesäureester-derivaten nach Verfahren a

2,6 g (0,007 Mol) des gemäß Abschnitt c) hergestellten 4-(3-Butenyl)benzoesäure-(4-propylcyclohex-1-en-1-yl)phenylesters wurden in 4,5 ml Methylenchlorid gelöst, 1 g (0,007 Mol) 2,2,4-Trimethyl-2,4-disilapentan sowie 0,2 ml einer 0,5 %igen Lösung von Dicyclopentadienylplatindichlorid (entsprechend 100 ppm Pt) zugegeben und bei Raumtemperatur gerührt. Nach Rühren über Nacht wurde eingeengt und der Rückstand an einer Kieselgelsäule mit Petrolether/Essigsäureethylester im Verhältnis 9:1 chromatographiert. Durch Fraktionierung wurden 2,2 g (60,4 % der Theorie) eines hochreinen Produkts erhalten, das folgendes Phasenverhalten aufweist: C 45 S_{C} 86 I.
C₃₂H₄₈O₂Si₂ (520,7)
(CH₃)₃Si-CH₂-Si(CH₃)₂-(CH₂)₄-C₆H₄-COO-C₆H₄-C₆H₇-(CH₂)₂-CH₃.

In analoger Weise wurden hergestellt:
1. 4-(6,6,8,8-Tetramethyl-6,8-disilanonyl)benzoesäure-[4-(4-methoxyphenylethyl)phenyl]ester, C₃₃H₄₆O₃Si₂, C (N 54) 60 I.
2. 4-[4-(5,5,8,8,10,10-Hexamethyl-5,8,10-trisilaundecyl-1) phenylethyl]benzoesäure-(4-octyloxyphenyl)ester, C₄₃H₆₈O₃Si₃, S_{I} 38 S_{C} 72 I.
3. 4-(11,11,14,14,16,16-Hexamethyl-11,14,16-trisilaheptadecyl-1) benzoesäure-[4-(4-propylcyclohex-1-en-1-yl)biphenylyl]ester, C₄₈H₇₄O₂Si₃, S_{G} 37 S_{F} 152 S_{C} 214 I.
4. 4-[4-(5,5,8,8,10,10-Hexamethyl-5,8,10-trisilaundecyl-1)phenylethyl]benzoesäurecholesterylester, C₅₆H₉₃O₂Si₃, C 103 S_{A} (76 S_{C}) 179 I.
5. 4-(5,5,8,8-Tetramethyl-5,8-disilanonyl-1)benzoesäure-[4-(4-propylcyclohex-1-en-1-yl)phenyl]ester, C₃₃H₅₀O₂Si₂, C 73 S_{E} 77 S_{C} 84 I.
6. 4-[4-(5,5,8,8,11,11-Hexamethyl-5,8,11-trisiladodecyl-1)phenylethyl]benzoesäurecholesterylester, C₅₇H₉₅O₂Si₃, C 90 S_{A} (86 S_{C}) 182 I.
7. 4-(7,7,9,9-Tetramethyl-7,9-disiladecyl)benzoesäure-[4-(4-butylphenyl)cyclohexyl]ester, C₃₅H₅₆O₂Si₂, -11 C 20 S_{B} 53 I
8. 4-(5,5,7,7,9,9-Hexamethyl-5,7,9-trisiladecyl-1)benzoesäure[4-(4-propylcyclohex-1-en-1-yl)phenyl]ester, C₃₅H₅₆O₂Si₃, S_{G} 43 S_{C} 71 I.
9. 4-(7,7,9,9-Tetramethyl-7,9-disiladecyl)benzoesäure-[4-(4-butylcyclohexyl)phenyl]ester, C₃₅H₅₆O₃Si₂, C 49 S_{C} 54 N 58 I.
10. 4-(10,10,12,12-Tetramethyl-10,12-disilatetradecyl-1-oxy)benzoesäure-[4-(4-chlorphenyl-ethyl)biphenylyl)ester, C₄₂H₅₅ClO₃Si₂. S_{E} 172 S_{B} 217 S_{A} 251 I.
11. 4-[10-(1-Methylcyclohex-1-en-4-yl)-5,5,8,8-tetramethyl-5,8-disilaundecyl-1]benzoesäure-[4-(4-propylcyclohex-1-en-1-yl)-phenyl]ester, C₄₂H₆₃O₂Si₂, C (S_{C} 40) 47 I.
12. 4-[5,5,7,7,10,10-Hexamethyl-6-oxa-5,7,10-trisilaundecycl-1]-benzoesäure-[4-(4-propylcyclohex-1-en-1-yl)phenyl]ester, C₃₅H₅₆O₃Si₃, C 28 S_{C} 72 I.
13. 4-[4-(5,5,7,7-Tetramethyl-5,7-disilaoctyl-1)phenylethyl]benzoesäure-(4-octyloxyphenyl)ester, C₃₉H₅₈O₃Si₂, C (S_{G}44S_{C}) 51S_{C} 84 I.
14. 4-[4-(5,5,7-Trimethyl-7-phenyl-5,7-disilaoctyl)phenylethyl]-benzoesäure-(4-octyloxyphenyl)ester, C₄₄H₆₀O₃Si₂, S_{I} 29 S_{C} 53 I.
15. 4-[4-(5,5,9,9-Tetramethyl-5,9-disiladecyl)phenylethyl]benzoesäure-(4-octyloxyphenyl)ester, C₄₁H₆₂O₃Si₂, S_{G} 44 S_{C} 83 I.
16. 4-(5,5,8,10,10-Pentamethyl-8-trimethylsilylmethyl-5,8,10-trisilaundecyl)benzoesäure-[4-(4-propylcyclohex-1-en-1-yl)phenyl]ester, C₃₉H₆₆O₂Si₄, S_{G} 45 S_{C} 55 I.
17. 4-(5,5,7,7-Tetramethyl-5,7-disilaoctyl)benzoesäure-[4-(4-chlorphenyl)ethylphenyl]ester, C₃₁H₄₁ClO₂Si₂, C (S_{E} 77 S_{B}) 80 S_{B}121 I.
18. 4-(5,5,7,7-Tetramethyl-5,7-disilaoctyl)benzoesäure-[4-(4-nonylcyclohexyl)phenyl]ester, C₃₈H₆₂O₂Si₂, C 80 S_{B} 82 I.
19. 4-[4-(5,5,7,7-Tetramethyl-5,7-disilaoctyl)phenylethyl]benzoesäure-(S)-(+)-octyl-2-ester, C₃₃H₅₀O₂Si₂, bei 20°C isotrop.
20. 4-(7,7,9,9-Tetramethyl-7,9-disiladecyloxy)benzoesäure-(4-propyl-bicyclo-[2.2.2]octyl-1)ester, C₃₀H₅₂O₃Si₂, C 63 N 66 I.
21. 4-(5,5,7,7-Tetramethyl-5,7-disilaoctyl)benzoesäure-4'-cyanobiphenylylester, C₃₀H₃₇NO₂Si₂, C 72 S_{A} 180 I.
22. 4-(7,7,9,9-Tetramethyl-7,9-disiladecyloxy)benzoesäure-(4'-propyl)-bicyclohexylester, C₃₄H₆₀O₃Si₂, C (S_{F} 32 S_{B}) 49 S_{B} 74 S_{A} 106 N 111 I.

### Beispiele für die Silanherstellung:

### Herstellung von 2,4,4-Trimethyl-2,4-disilapentan, (CH₃)₃SiCH₂-Si(CH₃)₂H:

Aus 2 ml käuflichem Chlormethyltrimethylsilan (Fa. Janssen, 4057 Brüggen) und 15 g (0,62 Mol) Magnesium in Tetrahydrofuran wurde mit Jodethan bei 60°C eine Grignardreaktion gestartet, nach deren Anspringen die Restmenge von insgesamt 73,6 g (0,6 Mol) des Silans, gelöst in 150 ml THF, in 3 Std. bei 60 - 70°C zugetropft wurde. Nach Abkühlen und Dekantieren wurde diese Lösung bei 15°C unter Rühren zu einer Lösung von 51 g (0,54 Mol) Chlordimethylsilan in 150 ml THF getropft, dann 2 Stunden bei Raumtemperatur gerührt, vom Niederschlag abfiltriert und das Filtrat auf Eis gegossen. Nach Ansäuern mit Salzsäure und Ausschütteln mit Methyl-tert.butylether wurde die entstandene organische Phase in üblicher Weise aufgearbeitet. Die Fraktionierung des eingeengten Rückstands ergab bei 112 - 116°C 69,3 g (87,6 % d. Th.) des gesuchten Silans.

### Herstellung von 2,5,5,7,7-Pentamethyl-2,5,7-trisilaoctan (CH₃)₃SiCH₂Si(CH₃)₂-CH₂-CH₂-Si(CH₃)₂H:

Analog dem vorstehenden Absatz wurde aus 68 g (0,55 Mol) Chlormethyltrimethylsilan in 150 ml THF eine Grignardlösung hergestellt, die dann bei 10°C zu einer Lösung von ebenfalls käuflichem Chlordimethylvinylsilan (ABCR GmbH, 7500 Karlsruhe) zugetropft wurde (2 Std.). Danach wurde auf Raumtemperatur erwärmt und schließlich noch 1 Stunde bei 35°C gehalten, wobei ein voluminöser Niederschlag entstand. Durch Hydrolyse, Ausschütteln mit Methyl-tert.butylether, Phasentrennung und -aufarbeitung wurden 106 g Rohprodukt erhalten, das bei der Fraktionierung bei 149 - 151°C 68 g (entsprechend 72 % d. Th.) an 3,3,5-Trimethyl-3,5-disilahex-1-en (C₈H₂₀Si₂) ergab.

Zu 68 g (0,39 Mol) dieses Silans wurden bei 60°C in Gegenwart von Platinkatalysator ( 100 ppm Pt) unter Rühren 50 g (0,53 Mol) Chlordimethylsilan getropft. Durch die Reaktionswärme stieg die Gemischtemperatur bis 85°C und wurde dabei gehalten. Nach 30 Minuten Nachreaktion bei 80°C wurde fraktioniert. Bei 16 hPa und 108 - 112°C wurden 77,4 g (74,3 % der Theorie) an 2-Chlor-2,5,5,7,7-pentamethyl-2,5,7-trisilaoctan erhalten. C₁₀H₂₇ClSi₃.

Zu einer Suspension von 3,2 g (0,084 Mol) LiAlH₄ in 150 ml absolutem Ether wurden innerhalb von 3 Stunden unter Rühren bei 10 - 30°C 77,4 g (0,29 Mol) des im vorigen Abschnitt beschriebenen Chlorsilans zugetropft. Nach 4 Stunden Nachreaktion bei Raumtemperatur wurde das überschüssige Alanat vorsichtig mit Wasser zersetzt, das Gemisch mit 10 ml 5 n NaOH basisch gemacht und die Phasen getrennt. Nach Aufarbeitung der Etherphase ergab der destillierte Rückstand bei 134 Pa und 82 - 83°C 62,5 g (92,9 % d. Th.) an 2,5,5,7,7-Pentamethyl-2,5,7-trisilaoctan. C₁₀H₂₈Si₃.

Durch analoge Verfahrensweisen wurden folgende Silane hergestellt:

2,5,5-Trimethyl-2,5-disilahexan, Kp. 129°C, C₇H₂₀Si₂.

2,5,5,8,8-Pentamethyl-2,5,8-trisilanonan, Kp. bei 5 hPa 78 - 80°C, C₁₁H₃₀Si₃.

6-(4-Methylcyclohex-3-en-1-yl)-4,4,6-trimethyl-2,4-disilaheptan, Kp. bei 8 hPa 130 - 131°C, C₁₆H₃₄Si₂.

2,6,6-Trimethyl-2,6-disilaheptan, Kp. bei 16 hPa 50°C, C₈H₂₂Si₂.

2,2,4,6,6-Pentamethyl-2,4,6-trisilaheptan, Kp. bei 16 hPa 66°C, C₉H₂₆Si₃.

2,4,4,7,7-Pentamethyl-3-oxa-2,4,7-trisilaoctan, Kp. bei 8 hPa 60°C, C₉H₂₆OSi₃.

2,4-Dimethyl-4-phenyl-2,4-disilapentan, Kp. bei 16 hPa 101 - 102°C, C₁₁H₂₀Si₂.

2,5,8,8-Tetramethyl-5-(trimethylsilylmethyl)-2,5,8-trisilaoctan, Kp. bei 5 hPa 96 - 97°C,
C₁₃H₃₆Si₄ = [(CH₃)₃SiCH₂]₂Si(CH₃)-CH₂CH₂-Si(CH₃)₂H

### Beispiel 2

### Vergleichsbeispiel a

Durch Umsetzung von 4-(3-Butenyl)benzoesäure-(4-propylcyclohex-1-en-1-yl)phenylester (vgl. Beispiel 1, Beispiele für ungesättigte Ester) mit Pentamethyldisiloxan mit Hilfe von Platinkatalysator in der in Beispiel 1 beschriebenen Weise läßt sich 4-[4-(Pentamethyldisiloxanyl)butyl]benzoesäure-[4-(4-propylcyclohex-1-en-1-yl)phenyl]ester herstellen (C₃₁H₄₆O₃Si₂), der folgendes Phasenverhalten aufweist:

C (50 S_{C}) 65 S_{G} 88 S_{C} 93 I.

Gegenüber der vollständig analogen erfindungsgemäßen, in Beispiel 1 Abschnitt d) zuerst beschriebenen Verbindung, in der nur der Sauerstoff zwischen den beiden Si-Atomen durch eine CH₂-Gruppe ersetzt ist, liegt hier der Schmelzpunkt um 20°C höher und die Phasenbreite der smektisch-C-Phase beim Aufheizen beträgt nur 5°C gegenüber 41°C bei der erfindungsgemäßen Verbindung.

### Vergleichsbeispiel b

Analog Beispiel 1, Abschnitt d), wurde die nicht erfindungsgemäße Verbindung 4-(4-Trimethylsilylbutyl)benzoesäure-4'-cyanobiphenylylester hergestellt, mit folgendem Phasenverhalten:

C 104 S_{A} 191 I.

Diese Verbindung weist im Gegensatz zu Verbindung 21 von Beispiel 1, Abschnitt d) nur den nicht erfindungsgemäßen Trimethylsilylrest auf, ist sonst identisch.

Der Schmelzpunkt liegt bei der erfindungsgemäßen Verbindung um 32°C niedriger und die Verbindung weist eine um 21° höhere Phasenbreite auf.

### Beispiel 3

### Verfahrensbeispiel (Verfahren c)

33,3 g (0,2 Mol) nach Beispiel 1 (Abschnitt b) hergestelltes 4-(3-Butenyl)-1-chlorbenzol wurde mit 2,4,4-Trimethyl-2,4-disilapentan (vgl. Beispiel 1 - Abschnitt d) mit Pt als Katalysator zu 1-Chlor-4-(5,5,7,7-tetramethyl-5,7-disilaoctyl-1)benzol silyliert. Der Siedepunkt bei 0,05 hPa lag bei 115°C, die Ausbeute betrug 70,8 % d. Th.

In gleicher Weise, wie in Beispiel 1 (Abschnitt b) beschrieben, wurde aus dem vorstehenden Si-Alkyl-chlorbenzol über eine Grignardverbindung durch Umsetzung mit CO₂ 4-(5,5,7,7-Tetramethyl-5,7-disilaoctyl)benzoesäure hergestellt (Ausbeute 51 % d. Th.) mit einem Schmelzpunkt von 112 - 113°C. C₁₇H₃₀Si₂O₂.

Durch Umsetzung dieser Säure mit Thionylchlorid in Toluol als Lösungsmittel wurde in 89,6 % Ausbeute 4-(5,5,7,7-Tetramethyl-5,7-disilaoctyl)benzoylchlorid (Siedepunkt bei 0,05 hPa 165°C) erhalten (C₁₇H₂₉ClOSi₂).

3,9 g (0,02 Mol) 4'-Cyanobiphenylol und 2 g Triethylamin wurden in 90 ml Toluol bei 90°C gelöst, 6,8 g (0,02 Mol) des obigen Säurechlorids zugegeben und das Gemisch 90 Minuten bei 100°C gerührt. Danach wurde abgekühlt, filtriert und eingeengt. Das Rohprodukt wurde aus Ethanol/Methanol (1:1) umkristallisiert (Ausbeute 70 %). Die Substanz (C₃₀H₃₇NO₂Si₂) zeigte folgendes Phasenverhalten: C 72 S_{A} 180 I.

### Beispiel 4

### Verfahrensbeispiel (Verfahren b)

Aus 80,5 g (0,5 Mol) 4-Chlorbenzylchlorid, gelöst in 450 ml Diethylether, wurde, beginnend mit einer Startmenge von 10 ml, mit 13,4 g (0,55 Mol) Magnesium eine Grignardreaktion in Gang gesetzt, die dann bei 10-15°C in 3 Stunden zu Ende geführt wurde. Die dekantierte Lösung wurde bei Raumtemperatur zu einer Lösung von 125 g (0,5 Mol) 4-Toluolsulfonsäure-(3-chlorpropyl)ester (durch einfache Veresterung des käuflichen 3-Chlorpropanols zu erhalten; Kp. bei 0,02 hPa 156°C) zugetropft und anschließend noch 2 Stunden auf 60°C erhitzt. Nach Abkühlung wurde hydrolysiert und aufgearbeitet. Durch Fraktionierung des organischen Anteils wurden bei 16 hPa und 166-168°C 55 g an 1-Chlor-4-(4-chlorbutyl)benzol erhalten. Von 40,6 g (0,2 Mol) dieses Produkts und 5,3 g (0,22 Mol) Magnesium wurde eine zweite Grignardlösung hergestellt, die dann bei 30-50°C mit 3,6 g (0,2 Mol) Si-Chlor-penta-Si-methyl-Si,Si'-methandiylbissilan (beispielsweise herstellbar nach Kumada et al., J. Org. Chem. 23, 292 (1958); Kp. 16 hPa 41°C) umgesetzt wurde. Dabei wurde in 65 % Ausbeute 1-Chlor-4-(5,5,7,7-tetramethyl-5,7-disilaoctyl-1)benzol erhalten (Kp. bei 0,05 hPa 115°C).

Dieses Produkt ist mit dem ersten Zwischenprodukt aus Beispiel 3 identisch und kann in gleicher Weise wie dort zu 4-(5,5,7,7-Tetramethyl-5,7-disilaoctyl)benzoesäure und diese dann weiter zu erfindungsgemäßen Verbindungen umgesetzt werden.

### Beispiel 5

### Herstellung von 3-Fluor-4-[2-(S)-methylbutoxy]benzoesäure-[4-(4-(5,5,7,7-tetramethyl-5,7-disilaoctyl)phenyl)ethylphenyl]ester 4-(C₂H₅CH(CH₃)-CH₂O)-3-F-C₆H₃COO-C₆H₄-(CH₂)₂-C₆H₄-(CH₂)₄-Si(CH₃)₂-CH₂-Si(CH₃)₃ nach Verfahren a:

4,5 g (0,02 Mol) 3-Fluor-4-[2-(S)-methylbutoxy]benzoesäure (beispielsweise herstellbar nach EP-A 255 962), 5,0 g (0,02 Mol) 4-[4-(3-Butenyl)phenylethyl]phenol, 4,1 g (0,02 Mol) Dicyclohexylcarbodiimid und 0,1 g 4-Dimethylaminopyridin wurden bei Raumtemperatur in 40 ml Ether zusammengegeben. Das Gemisch wurde erst 4 Stunden auf Rückfluß erhitzt, dann 14 Stunden bei Raumtemperatur gerührt, der entstandene Harnstoff abfiltriert, erst mit 2 n Salzsäure, dann mit NaHCO₃-Lösung gewaschen und die Etherphase aufgearbeitet. Durch Säulenchromatographie an Al₂O₃ mit Methyltert.Butylether/Petrolether (1:2) als Laufmittel wurden 4,9 g 3-Fluor-4-(2-(S)-methyl-butoxy)benzoesäure-[4-(4-(3-butenyl)-phenyl)ethylphenyl]ester erhalten (Ausbeute 53,9 %), der folgendes Phasenverhalten zeigte: C 77 N* 86 I.

Durch Hydrosilylierung dieses Esters mit 2,4,4-Trimethyl-2,4-disilapentan unter Pt-Katalyse gemäß Beispiel 1 d wurde die Titel-Verbindung erhalten (C₃₆H₅₁FO₃Si₂; 56,1 % Ausbeute), die dann gegenüber der unsilylierten Verbindung folgende Phasen zeigte:

G -44 S_{C}* 38 I.

### Beispiel 6

### Herstellung von 4-(5,5,7,7-Tetramethyl-5,7-disilaoctyl)benzoesäure-4-(6-ethylpyridyl-2)ethenylphenyl)ester, nach Verfahren c.

Zur Herstellung des benötigten Pyridylethenylphenols wurden 72,6 g (0,6 Mol) 2-Methyl-5-ethylpyridin und 73,2 g (0,6 Mol) 4-Hydroxybenzaldehyd in 125 ml Acetanhydrid 20 Stunden auf 155°C erhitzt, abgekühlt und in ein Gemisch aus 300 ml Methanol und 60 ml konzentrierter Salzsäure geschüttet. Nach 3 Stunden Rückflußkochen (62°C) wurde erneut abgekühlt, 120 ml 25 %iger Ammoniak und 10 g Natriumacetat zugefügt und das ausgefallene Produkt abfiltriert. Nach Umkristallisieren aus 1,25 1 Ethanol wurden 42,8 g an Produkt erhalten (31,6 % der Theorie), das bei 227°C schmolz. C₁₅H₁₅NO.

5,8 g (0,017 Mol) 4-(5,5,7,7-Tetramethyl-5,7-disilaoctyl)benzoesäurechlorid (Herstellung in Beispiel 3 beschrieben) wurden bei 20°C zu 3,4 g (0,015 Mol) 5-Ethyl-2-[(4-hydroxyphenyl)ethenyl]-pyridin und 2,1 ml Triethylamin in 350 ml Toluol/Tetrahydrofuran (1:2,5) zugetropft. Nach 3 Stunden Rühren wurde noch 4 Stunden auf Rückfluß erhitzt.

Nach Erkalten, Abfiltrieren des Niederschlags und Einengen wurde der Rückstand aus Ethanol, dann noch zweimal aus Methanol umkristallisiert. Erhalten wurden 4,7 g (59,5 % der Theorie) der Titelverbindung, mit folgendem Phasenverhalten: C 64 S_{I} 102 N 105 I. 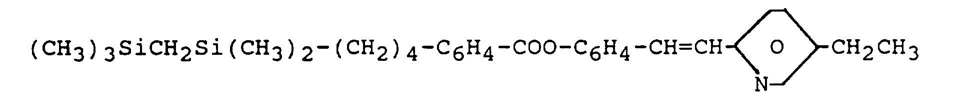 Diese Verbindung konnte nicht durch entsprechende Silylierung des analogen 4-(3-Butenyl)-benzoesäureesters erhalten werden, da der Pyridyl-Stickstoff den Pt-Katalysator blockierte.

### Beispiel 7

### Herstellung von 4-(5,5,7,7-Tetramethyl-5,7-disilaoctyl)benzoesäure-[4-(5-propyl-1,3-dioxan-2-yl)phenyl]ester, nach Verfahren a.

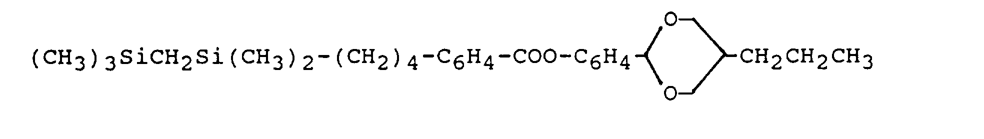

Käuflicher 2-Propylmalonsäurediethylester wurde in bekannter Weise bei 25 - 30°C mit Lithiumaluminiumhydrid zu 3-Hydroxymethyl-pentanol reduziert. Käuflicher 4-Hydroxybenzaldehyd wurde mit 4-(3-Butenyl)benzoesäurechlorid (Herstellung in Beispiel 1 beschrieben) bei 20 - 30°C unter Verwendung von Triethylamin als Hilfsbase verestert. Der so hergestellte 4-(3-Butenyl)benzoesäure-(4-formylphenyl)ester (C₁₈H₁₆O₃) schmolz bei 49°C.

14 g (0,05 Mol) dieses Formylesters und 5,9 g (0,05 Mol) des oben genannten Diols wurden mit 0,1 ml Schwefelsäure in 150 ml Toluol auf Rückfluß erhitzt und das entstehende Reaktionswasser azeotrop abdestilliert. Nach Abkühlen, Neutralisieren, Waschen und Einengen wurde ein Rohprodukt erhalten, das nach Umkristallisieren aus n-Heptan folgende Phasen zeigte: C 100 N 182 I. Die Ausbeute betrug 54,7 % d. Th.

4,57 g (0,012 Mol) dieses Produkts wurden mit 2,02 g (0,015 Mol) an 2,2,4-Trimethyl-2,4-disilapentan in 10 ml Methylenchlorid mit 0,4 ml an 0,5 %iger Dicyclopentadienylplatin-dichloridlösung bei Raumtemperatur zusammengegeben. Innerhalb 60 Minuten erwärmte sich das Gemisch auf 30°C, das noch 12 Stunden weitergerührt wurde. Der eingeengte Rückstand wurde an Kieselgel chromatographiert. Die Ausbeute betrug 61,3 % d. Th., C₃₀H₄₆O₄Si₂. Die Verbindung zeigte folgendes Phasenverhalten: C (56 N) 71 I.

### Beispiel 8

### Herstellung von 4-(6,6,8,8-Tetramethyl-6,8-disilanonyl)benzoesäure-[4-(5-hexylpyrimidin-2-yl)phenyl]ester nach Verfahren a:

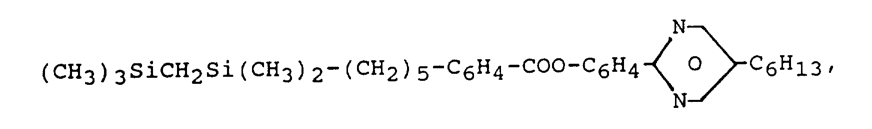

C₃₅H₅₀N₂O₂Si₂

4-(4-Pentenyl)benzoesäurechlorid wurde mit 5-Hexyl-2-(4-hydroxyphenyl)pyrimidin mit Triethylamin als Hilfsbase verestert. Der erhaltene (62,5 % Ausbeute) 4-(4-Pentenyl)benzoesäure-[4-(5-hexylpyrimidin-2-yl)phenyl]ester (C₂₈H₃₄N₂O₂) zeigte folgendes Phasenverhalten: C 52 N 139 I.

3 g (0,007 Mol) dieses Esters wurden mit 1,1 g (0,0075 Mol) 2,4,4-Trimethyl-2,4-disilapentan (Herstellung in Beispiel 1 beschrieben) unter Verwendung von 5 ml einer 0,5 %igen Lösung von Dicyclopentadienylplatindichlorid in 12 ml Dichlormethan in der ebenfalls in Beispiel 1 beschriebenen Weise bei 60°C hydrosilyliert. Durch Chromatographie des Rohprodukts an Kieselgel mit Petrolether/Dichlormethan (1:2) wurde das Titelprodukt rein erhalten, das folgendes Phasenverhalten aufweist: C 41 S_{C} 44 S_{A}, N 48 N 67 I.

### Herstellung von 2-[4-(5,5,7,7-Tetramethyl-5,7-disilaoctyloxy)phenyl]5-hexylpyrimidin nach Verfahren c

Durch Umsetzung des vorstehend beschriebenen Pyridinderivats (Na-Salz) mit dem Toluolsulfonsäureester des 5,5,7,7-Tetramethyl-5,7-disilaoctanols wurde die Titelverbindung erhalten: C₂₆H₄₄N₂OSi₂. Sie zeigte folgendes Phasenverhalten: C 25 N 48 I.

### Beispiel 9

### Herstellung von 4'-(10,10,12,12-Tetramethyl-10,12-disilatridecyloxy)biphenylcarbonsäure-1-(S)-(-)-(1-cyano-2-methylpropyl-1)-ester nach Verfahren c

Eine Lösung von 4,5 g (0,022 Mol) N,N'-Dicyclohexylcarbodiimid in 30 ml Dichlormethan wurde zu einer Lösung von 9,6 g (0,02 Mol) 4'-(10,10,12,12-Tetramethyl-10,12-disilatridecyloxy)biphenyl-4-carbonsäure und 4,1 g (0,02 Mol) 4-N-Pyrrolidinopyridin (=N-PPY) in 110 ml Dichlormethan getropft und das Gemisch 18 Stunden bei Raumtemperatur gerührt. Danach wurde der entstandene Harnstoff abfiltriert und das Rohprodukt durch Chromatographie an Kieselgel mit Dichlormethan/Heptan (4:1) gereinigt. Dieser Ester (90 % Ausbeute) wurde nun nach L. K. M. Chan et al. (Mol. Cryst. Liq. Cryst. 1989, 172, 125) durch Hydrierung gespalten. Die so entstandene freie Säure wurde mit Oxalylchlorid/Ammoniak in das entsprechende Amid überführt. Die Umsetzung mit Thionylchlorid in DMF lieferte die Titelverbindung. Phasenverhalten: C 45 I.

## Patentansprüche

1. Verbindungen der allgemeinen Formel I
M-(CH₂)ₙ-(O)ₘ-[-D-B-]_{q}-Y , (I)
in der bedeuten
**M** einen Rest aus 2 bis 5 Siliciumatomen in linearer, verzweigter oder cyclischer Anordnung, die untereinander mit Brückenelementen A verbunden sind, wobei die übrigen Valenzen der Siliciumatome mit Resten R abgesättigt sind;
**A** C₁- bis C₈-Alkylenreste oder Sauerstoff als Brückenelemente, mit der Maßgabe, daß je Rest M mindestens ein Rest der Bedeutung A einen C₁- bis C₈-Alkylenrest bedeutet;
**R** gleiche oder verschiedene, gegebenenfalls mit Fluor- oder Chloratomen oder Cyanogruppen substituierte geradkettige C₁- bis C₁₀-Alkyl- oder C₂- bis C₁₀-Alkenylreste, verzweigtkettige C₃- bis C₁₀-Alkyl- oder C₃- bis C₁₀-Alkenylreste, gegebenenfalls mit C₁- bis C₄-Alkyl-, C₁- bis C₄-Alkoxyresten, Fluor-, Chlor-, Bromatomen, Cyano-, Trifluormethyl- oder Nitroresten substituierte C₆- bis C₁₂-Cycloalkyl-, C₆- bis C₁₂-Cycloalkenyl-, C₆- bis C₁₂-Alkylcycloalkyl-, C₆- bis C₁₂-Alkylcycloalkenyl-, C₆- bis C₁₂-Aryl- oder C₆- bis C₁₂-Aralkylreste;
**n** eine ganze Zahl von 3 bis 12;
**m** 0 oder 1;
**D** gleiche oder verschiedene isocyclische oder heterocyclische gesättigte oder ungesättigte 5- oder 6-gliedrige Ringe;
**B** gleiche oder verschiedene Bindeglieder, die ausgewählt werden aus einer chemischen Bindung, einer Gruppe -COO-, -OOC-, -CH₂-CH₂-, -CH=CH-, -C≡C-, -CH=N-, -N=CH-, -CH₂-O-, -O-CH₂- und -N=N-;
**q** eine ganze Zahl von 1 bis 3;
**Y** ein Wasserstoffatom oder eine geradkettige oder verzweigtkettige C₁- bis C₁₀-Alkyl- oder C₁- bis C₁₀-Alkoxygruppe oder einen mit einem Wasserstoffatom versehenen oder substituierten Rest der Bedeutung D, wobei gegebenenfalls 1 bis 2 vorkommende Ring-Substituenten C₁- bis C₄-Alkyl-, C₁- bis C₄-Alkoxyreste, Fluor-, Chlor- oder Bromatome, Cyano-, Trifluormethyl- oder Nitroreste sein können, oder einen Cholesterylrest.

2. Verbindungen nach Anspruch 1, bei denen bedeuten:
**M** einen Rest aus 2 bis 5 Siliciumatomen in linearer oder verzweigter Anordnung, die untereinander mit Brückengliedern A verbunden sind, wobei die übrigen Valenzen der Siliciumatome mit Resten R abgesättigt sind;
**A** eine Methylen-, 1,2-Ethylen- oder 1,3-Propylengruppe oder ein Sauerstoffatom mit der Maßgabe, daß je Rest M mindestens ein Rest der Bedeutung A eine Methylen-, 1,2-Ethylen- oder eine 1,3-Propylengruppe bedeutet,
**R** gleiche oder verschiedene C₁-bis C₄-Alkylreste, wobei einer der am terminalen Siliciumatom befindlichen Reste ausgewählt wird aus geradkettigen C₁-bis C₁₀-Alkyl- oder Alkenylresten, mit Fluor- oder Chloratomen substituierten C₁- bis C₅-Alkylresten, verzweigten C₃-bis C₁₀-Alkyl- oder C₃- bis C₁₀-Alkenylresten, gegebenenfalls mit C₁- bis C₄-Alkyl-, C₁- bis C₄-Alkoxyresten, Fluor-, Chlor-, Bromatomen, Cyano-, Trifluormethyl- oder Nitroresten substituierten C₆- bis C₁₂-Cycloalkyl-, C₆- bis C₁₂-Cycloalkenyl-, C₆- bis C₁₂-Alkylcycloalkyl-, C₆- bis C12-Alkylcycloalkenyl-, C₆-bis C₁₂-Aryl- oder C₆- bis C₁₂-Aralkylresten;
**n** eine ganze Zahl von 3 bis 10;
**m** den Wert 0 oder 1,
**D** gleiche oder verschiedene Reste, die ausgewählt werden aus 1,4-Phenylenresten, 1,4-Cyclohexylidenresten, 2,5-oder 3,6-Pyrimidindiylreste, 2,5-Dioxandiylreste oder 1,4-Bicyclo[2.2.2]-octandiylreste;
**B** gleiche oder verschiedene Bindeglieder, die ausgewählt werden aus einer chemischen Bindung, einer -COO-, -OOC-, -CH₂-CH₂- oder einer -CH=CH-Gruppe;
**q** eine ganze Zahl von 1 bis 3;
**Y** ein Wasserstoffatom oder eine geradkettige oder verzweigtkettige C₁- bis C₁₀-Alkyl- oder C₁- bis C₁₀-Alkoxygruppe oder einen mit einem Wasserstoffatom versehenen oder substituierten Phenylrest oder mit einem Wasserstoffatom versehenen oder substituierten Cyclohexyl- oder Cyclohexenylrest, wobei gegebenenfalls 1 oder 2 vorkommende Substituenten C₁- bis C₄-Alkyl-, C₁- bis C₄-Alkoxyreste, Fluor- oder Chloratome oder Cyanoreste sein können oder einen Cholesterylrest.

3. Verbindungen nach Anspruch 1 oder 2, bei denen bedeuten:
**n** eine ganze Zahl von 4 bis 8;
**m** den Wert 0 oder 1;
**D** gleiche oder verschiedene Reste, die ausgewählt werden aus 1,4-Phenylen- oder 1,4-Cyclohexylidenresten;
**B** gleiche oder verschiedene Bindeglieder, die ausgewählt werden aus einer chemischen Bindung, einer -COO-, -OOC- oder einer -CH₂-CH₂-Gruppe;
**q** den Wert 1 oder 2;
**Y** ein Wasserstoffatom oder eine geradkettige oder verzweigtkettige C₁- bis C₁₀-Alkylgruppe oder C₁- bis C₁₀-Alkoxygruppe oder einen mit einem Wasserstoffatom versehenen oder substituierten Phenyl- oder Cyclohexylrest, wobei gegebenenfalls 1 oder 2 vorkommende Substituenten C₁- bis C₄-Alkyl-, C₁- bis C₄-Alkoxyreste, Fluor- oder Chloratome oder Cyanoreste sein können oder einen Cholesterylrest;
**M** einen Rest der allgemeinen Formel II:
R-[Si(CH₃)₂-A]ₚ-SiR₂-, (II)
in der bedeuten
**A** eine Methylengruppe;
**R** eine geradkettige C₁- bis C₄-Alkylgruppe oder einen Phenylrest;
**p** den Wert 1 oder 2.

4. Verfahren zur Herstellung von Verbindungen der allgemeinen Formel I gemäß einem der Ansprüche 1 bis 3 durch Umsetzung von Verbindungen der allgemeinen Formel MH mit Verbindungen der allgemeinen Formel III
H₂=CH-(CH₂)ₙ₋₂-(O)ₘ-[-D-B-]_{q}-Y (III)
in Gegenwart von mindestens einem Metall der Platingruppe und/oder dessen Verbindungen, wobei M, A, R, n, m, D, B, q, Y, und p die in den vorstehenden allgemeinen Formeln I und II in den Ansprüchen 1 bis 3 angegebenen Bedeutungen aufweisen, mit der Maßgabe, daß das Wasserstoffatom in MH an ein Siliciumatom gebunden ist.

5. Verfahren zur Herstellung von Verbindungen der allgemeinen Formel I gemäß einem der Ansprüche 1 bis 3 durch Umsetzung von Verbindungen der allgemeinen Formel M-**Hal**, in der Hal ein Chlor-, Brom- oder Iodatom bedeutet, mit Verbindungen der allgemeinen Formel IV
T-(CH₂)ₙ-(O)ₘ-[-D-B-]_{q}-Y , (IV)
in der **T** ein Alkalimetallatom oder Mg-Hal bedeutet, wobei Hal die vorstehenden Bedeutungen aufweist, unter Abspaltung eines Metallhalogenids T-Hal, wobei M, A, R, n, m, D, B, q, Y, und p die in den vorstehenden allgemeinen Formeln I und II in den Ansprüchen 1 bis 3 angegebenen Bedeutungen aufweisen, mit der Maßgabe, daß für den Fall, daß B und/oder Y mit Alkalimetallen oder Magnesium oder Verbindungen der allgemeinen Formel IV reagieren, B und/oder Y erst nach erfolgter Umsetzung von M-Hal mit Verbindungen der allgemeinen Formel IV eingeführt werden.

6. Verfahren zur Herstellung von Verbindungen der allgemeinen Formel I gemäß einem der Ansprüche 1 bis 3 durch Umsetzung von Verbindungen der allgemeinen Formel V
M-(CH₂)ₙ-(O)ₘ-[-D-B-]ᵣ₋₁-D-G (V)
mit Verbindungen der allgemeinen Formel VI
Q-[-D-B-]ₛ-Y (VI)
wobei **r** und **s** jeweils ganze Zahlen von 0 bis 3 sind, deren Summe q beträgt, **G** und **Q** jeweils einen der Reste -OH, -OLi, -ONa, -OK, -O-C₁- bis -O-C₄-Alkyl, -COOH, -COBr, -COCl, -NH₂, -O-tos oder -MgHal bedeuten und M, A, R, n, m, D, B, q, Y, und p die in den vorstehenden allgemeinen Formeln I und II in den Ansprüchen 1 bis 3 und Hal die in Anspruch 5 angegebenen Bedeutungen aufweisen, unter Bildung von Bindegliedern B und unter Abspaltung von Verbindungen **W** mit der Bedeutung Wasser, C₁- bis C₄-Alkanol, HHal, MgHal₂, LiHal, NaHal, KHal, Li-O-tos, Na-O-tos oder K-O-tos.

7. Flüssigkristalline Zusammensetzungen, die mindestens eine Verbindung gemäß einem der Ansprüche 1 bis 3 oder nach dem Verfahren gemäß einem der Ansprüche 4 bis 6 herstellbare Verbindung umfassen und die nematische, smektische und/oder cholesterische Phasen aufweisen.

8. Verwendung der Verbindungen gemäß einem der Ansprüche 1 bis 3 oder der nach dem Verfahren gemäß einem der Ansprüche 4 bis 6 herstellbaren Verbindungen und der flüssigkristallinen Zusammensetzungen gemäß Anspruch 7 in der Opto-Elektronik, Informationsspeicherung, Signalgebung oder in elektrographischen Verfahren.

## Claims

1. Compounds of the general formula I
M-(CH₂)ₙ-(O)ₘ-[-D-B-]_{q}-Y, (I)
in which
M denotes a radical composed of 2 to 5 silicon atoms in linear, branched or cyclic arrangement, which are linked together by bridging elements A, the remaining valencies of the silicon atoms being saturated with radicals R;
A denotes C₁- to C₈-alkylene radicals or oxygen as bridging elements, with the proviso that at least one radical of the meaning A per M radical denotes a C₁- to C₈-alkylene radical;
R denotes identical or different, optionally fluorine or chlorine atom- or cyano group-substituted straight-chain C₁- to C₁₀-alkyl or C₂- to C₁₀-alkenyl radicals, branched chain C₃- to C₁₀-alkyl or C₃- to C₁₀-alkenyl radicals, optionally C₁- to C₄-alkyl-, C₁- to C₄-alkoxy-radical-, fluorine, chlorine, bromine atom-, cyano, trifluoromethyl or nitro radical-sub-stituted C₆- to C₁₂-cycloalkyl, C₆- to C₁₂- cycloalkenyl, C₆- to C₁₂-alkylcycloalkyl, C₆- to C₁₂-alkylcycloalkenyl, C₆- to C₁₂-aryl or C₆- to C₁₂-aralkyl radicals;
n denotes an integer from 3 to 12;
m denotes 0 or 1;
D denotes identical or different isocyclic or heterocyclic saturated or unsaturated 5- or 6-membered rings;
B denotes identical or different linkers selected from a chemical bond, a group -COO-, -OOC-, -CH₂-CH₂-, -CH=CH-, -C≡C-, -CH=N-, -N=CH-, -CH₂-O-, -O-CH₂- and -N=N-;
q denotes an integer from 1 to 3;
Y denotes a hydrogen atom or a straight-chain or branched chain C₁- to C₁₀-alkyl or C₁- to C₁₀-alkoxy group or a radical of the meaning D which is provided with a hydrogen atom or substituted, it being possible, where appropriate, for 1 to 2 ring substituents which occur to be C₁- to C₄-alkyl, C₁-to C₄-alkoxy radicals, fluorine, chlorine or bromine atoms, cyano, trifluoromethyl or nitro radicals, or denotes a cholesteryl radical.

2. Compounds according to Claim 1, in which:
M denotes a radical composed of 2 to 5 silicon atoms in linear or branched arrangement, which are linked together by bridging members A, the remaining valencies of the silicon atoms being saturated with radicals R;
A denotes a methylene, 1,2-ethylene or 1,3-propylene group or an oxygen atom with the proviso that at least one radical of the meaning A per radical M denotes a methylene, 1,2-ethylene or a 1,3-propylene group,
R denotes identical or different C₁- to C₄-alkyl radicals, where one of the radicals located on the terminal silicon atom is selected from straight-chain C₁- to C₁₀-alkyl or alkenyl radicals, fluorine or chlorine atom-substituted C₁- to C₅-alkyl radicals, branched C₃- to C₁₀-alkyl or C₃- to C₁₀-alkenyl radicals, optionally C₁- to C₄-alkyl-, C₁-to C₄-alkoxy-radical-, fluorine, chlorine, bromine atom-, cyano, trifluoromethyl or nitro radical-substituted C₆- to C₁₂-cycloalkyl, C₆- to C₁₂-cycloalkenyl, C₆- to C₁₂-alkylcycloalkyl, C₆- to C₁₂-alkylcycloalkenyl, C₆- to C₁₂-aryl or C₆- to C₁₂-aralkyl radicals;
n denotes an integer from 3 to 10;
m denotes the value 0 or 1,
D denotes identical or different radicals selected from 1,4-phenylene radicals, 1,4-cyclohexylidene radicals, 2,5- or 3,6-pyrimidinediyl radicals, 2,5-dioxanediyl radicals or 1,4-bicyclo[2.2.2]octanediyl radicals;
B denotes identical or different linkers selected from a chemical bond, a -COO-, -OOC-, -CH₂-CH₂- or a -CH=CH- group;
q denotes an integer from 1 to 3;
Y denotes a hydrogen atom or a straight-chain or branched chain C₁- to C₁₀-alkyl or C₁- to C₁₀-alkoxy group or a phenyl radical which is provided with a hydrogen atom or substituted, or cyclohexyl or cyclohexenyl radical which is provided with a hydrogen atom or substituted, it being possible, where appropriate, for 1 or 2 substituents which occur to be C₁- to C₄-alkyl, C₁- to C₄-alkoxy radicals, fluorine or chlorine atoms or cyano radicals, or denotes a cholesteryl radical.

3. Compounds according to Claim 1 or 2, in which:
n denotes an integer from 4 to 8;
m denotes the value 0 or 1;
D denotes identical or different radicals selected from 1,4-phenylene or 1,4-cyclohexylidene radicals;
B denotes identical or different linkers selected from a chemical bond, a -COO-, -OOC- or a -CH₂-CH₂-group;
q denotes the value 1 or 2;
Y denotes a hydrogen atom or a straight-chain or branched chain C₁- to C₁₀-alkyl group or C₁- to C₁₀-alkoxy group or a phenyl or cyclohexyl radical which is provided with a hydrogen atom or substituted, it being possible, where appropriate, for 1 or 2 substituents which occur to be C₁- to C₄-alkyl, C₁- to C₄-alkoxy radicals, fluorine or chlorine atoms or cyano radicals, or denotes a cholesteryl radical;
M denotes a radical of the general formula II:
R-[Si(CH₃)₂-A]ₚ-SiR₂-, (II)
in which
A denotes a methylene group;
R denotes a straight-chain C₁- to C₄-alkyl group or a phenyl radical;
p denotes the value 1 or 2.

4. Process for the preparation of compounds of the general formula I according to any of Claims 1 to 3 by reacting compounds of the general formula MH with compounds of the general formula III
H₂=CH-(CH₂)ₙ₋₂-(O)ₘ-[-D-B-]_{q}-Y (III)
in the presence of at least one metal of the platinum group and/or of its compounds, where M, A, R, n, m, D, B, q, Y and p have the meanings stated in the abovementioned general formulae I and II in Claims 1 to 3, with the proviso that the hydrogen atom in MH is bonded to a silicon atom.

5. Process for the preparation of compounds of the general formula I according to any of Claims 1 to 3 by reacting compounds of the general formula M-Hal in which Hal denotes a chlorine, bromine or iodine atom with compounds of the general formula IV
T-(CH₂)ₙ-(O)ₘ-[-D-B-]_{q}-Y (IV)
in which T denotes an alkali metal atom or Mg-Hal, where Hal has the abovementioned meanings, with elimination of a metal halide T-Hal, where M, A, R, n, m, D, B, q, Y and p have the meanings stated in the abovementioned general formulae I and II in Claims 1 to 3, with the proviso that in the case where B and/or Y react with alkali metals or magnesium or compounds of the general formula IV, B and/or Y are introduced only after reaction of M-Hal with compounds of the general formula IV has taken place.

6. Process for the preparation of compounds of the general formula I according to any of Claims 1 to 3 by reacting compounds of the general formula V
M-(CH₂)ₙ-(O)ₘ-[-D-B-]ᵣ₋₁-D-G (V)
with compounds of the general formula VI
Q-[-D-B-]ₛ-Y (VI)
where r and s are each integers from 0 to 3 whose total is q, G and Q each denote one of the radicals -OH, -OLi, -ONa, -OK, -O-C₁- to -O-C₄-alkyl, -COOH, -COBr, -COCl, -NH₂, -O-tos or -MgHal, and M, A, R, n, m, D, B, q, Y and p have the meanings stated in the abovementioned general formulae I and II in Claims 1 to 3 and Hal has the meanings stated in Claim 5, to form linkers B and to eliminate compounds W with the meaning of water, C₁- to C₄-alkanol, HHal, MgHal₂, LiHal, NaHal, KHal, Li-O-tos, Na-O-tos or K-O-tos.

7. Liquid crystalline compositions which comprise at least one compound according to any of Claims 1 to 3 or compound which can be prepared by the process according to any of Claims 4 to 6 and which display nematic, smectic and/or cholesteric phases.

8. The use of the compounds according to any of Claims 1 to 3 or of the compounds which can be prepared by the process according to any of Claims 4 to 6 and of the liquid crystalline compositions according to Claim 7 in optoelectronics, information storage, signal generation or in electrographic processes.

## Revendications

1. Composés de formule générale I
M-(CH₂)ₙ-(O)ₘ-[-D-B-]_{q}-Y, (I)
dans laquelle
M représente un radical composé de 2 à 5 atomes de silicium disposés de façon linéaire, ramifiée ou cyclique, qui sont liés les uns aux autres par des éléments pontants A, les valences restantes des atomes de silicium étant saturées avec des radicaux R;
A représente des radicaux alkylène en C₁-C₈ ou oxygène comme éléments pontants, à condition qu'au moins un radical représenté par A, par radical M, représente un radical alkylène en C₁-C₈;
R représente des radicaux alkyle en Cₗ-C₁₀ ou alcényle en C₂-C₁₀ à chaîne droite, identiques ou différents, éventuellement substitués par des atomes de fluor ou de chlore ou par des groupes cyano, des radicaux alkyle en C₃-C₁₀ ou alcényle en C₃-C₁₀ à chaîne ramifiée, des radicaux cycloalkyle en C₆-C₁₂, cycloalcényle en C₆-C₁₂, alkylcycloalkyle en C₆-C₁₂, alkylcycloalcényle en C₆-C₁₂, aryle en C₆-C₁₂ ou arylalkyle en C₆-C₁₂, éventuellement substitués par des radicaux alkyle en C₁-C₄, alcoxy en C₁-C₄, des atomes de fluor, de chlore, de brome ou des radicaux cyano, trifluorométhyle ou nitro;
n représente un nombre entier de 3 à 12;
m représente 0 ou 1;
D représente des noyaux à 5 ou 6 chaînons, saturés ou insaturés, isocycliques ou hétérocycliques, identiques ou différents;
B représente des groupes de liaison, identiques ou différents, choisis parmi une liaison chimique, un groupe -COO-, -OOC-, -CH₂-CH₂-, -CH=CH-, -C≡C-, -CH=N-, -N=CH-, -CH₂-O-, -O-CH₂- et -N=N-;
q représente un nombre entier de 1 à 3;
Y représente un atome d'hydrogène ou un groupe alkyle en C₁-C₁₀ ou alcoxy en C₁-C₁₀, à chaîne droite ou à chaîne ramifiée, ou un radical représenté par D qui est muni d'un atome d'hydrogène ou substitué, avec la possibilité, le cas échéant, pour 1 ou 2 substituants du noyau présents d'être des radicaux alkyle en C₁-C₄, alcoxy en C₁-C₄, des atomes de fluor, de chlore ou de brome, des radicaux cyano, trifluorométhyle ou nitro, ou représente un radical cholestéryle.

2. Composés selon la revendication 1, dans lesquels:
M représente un radical composé de 2 à 5 atomes de silicium disposés de façon linéaire ou ramifiée, qui sont liés les uns aux autres par des éléments pontants A, les valences restantes des atomes de silicium étant saturées avec des radicaux R;
A représente un groupe méthylène, 1,2-éthylène ou 1,3-propylène, ou un atome d'oxygène, à condition qu'au moins un radical représenté par A, par radical M, représente un groupe méthylène, 1,2-éthylène ou 1,3-propylène;
R représente des radicaux alkyle en C₁-C₄ identiques ou différents, l'un des radicaux situés sur l'atome de silicium terminal étant choisi parmi des radicaux alkyle ou alcényle en C₁-C₁₀ à chaîne droite, des radicaux alkyle en C₁-C₅ substitués par des atomes de fluor ou de chlore, des radicaux alkyle en C₃-C₁₀ ou alcényle en C₃-C₁₀ ramifié, des radicaux cycloalkyle en C₆-C₁₂, cycloalcényle en C₆-C₁₂, alkylcycloalkyle en C₆-C₁₂, alkylcycloalcényle en C₆-C₁₂, aryle en C₆-C₁₂ ou aralkyle en C₆-C₁₂, éventuellement substitués par des radicaux alkyle en C₁-C₄, alcoxy en C₁-C₄, par des atomes de fluor, de chlore, de brome ou des radicaux cyano, trifluorométhyle ou nitro;
n représente un nombre entier de 3 à 10;
m représente la valeur 0 ou 1;
D représente des radicaux identiques ou différents, choisis parmi les radicaux 1,4-phénylène, les radicaux 1,4-cyclohexylidène, les radicaux 2,5- ou 3,6-pyrimidinediyle, les radicaux 2,5-dioxanediyle ou les radicaux 1,4-bicyclo[2,2,2]octanediyle;
B représente des groupes de liaison, identiques ou différents, choisis parmi une liaison chimique, un groupe -COO-, -OOC-, -CH₂-CH₂- ou un groupe -CH=CH-;
q représente un nombre entier de 1 à 3;
Y représente un atome d'hydrogène ou un groupe alkyle en C₁-C₁₀ ou alcoxy en C₁-C₁₀, à chaîne droite ou à chaîne ramifiée, ou un radical phényle qui est muni d'un atome d'hydrogène ou substitué, ou un radical cyclohexyle ou cyclohexényle qui est muni d'un atome d'hydrogène ou substitué, avec la possibilité, le cas échéant, pour 1 ou 2 substituants du noyau présents d'être des radicaux alkyle en C₁-C₄, alcoxy en C₁-C₄, des atomes de fluor ou de chlore ou des radicaux cyano, ou représente un radical cholestéryle.

3. Composés selon la revendication 1 ou 2, dans lesquels:
n représente un nombre entier de 4 à 8;
m représente la valeur 0 ou 1;
D représente des radicaux identiques ou différents, choisis parmi les radicaux 1,4-phénylène ou 1,4-cyclohexylidène;
B représente des groupes de liaison, identiques ou différents, choisis parmi une liaison chimique, un groupe -COO-, -OOC- ou -CH₂-CH₂-;
q représente la valeur 1 ou 2;
Y représente un atome d'hydrogène ou un groupe alkyle en C₁-C₁₀ ou un groupe alcoxy en C₁-C₁₀ à chaîne droite ou à chaîne ramifiée, ou un radical phényle ou cyclohexyle qui est muni d'un atome d'hydrogène ou substitué, avec la possibilité, le cas échéant, pour 1 ou 2 substituants du noyau présents d'être des radicaux alkyle en C₁-C₄, alcoxy en C₁-C₄, des atomes de fluor ou de chlore ou des radicaux cyano, ou représente un radical cholestéryle;
M représente un radical de formule générale II:
R-[Si(CH₃)₂-A]ₚ-SiR₂-, (II)
dans laquelle
A représente un groupe méthylène;
R représente un groupe alkyle en C₁-C₄ à chaîne droite ou un radical phényle;
p représente la valeur 1 ou 2.

4. Procédé de préparation de composés de formule générale I selon l'une quelconque des revendications 1 à 3, qui consiste à faire réagir des composés de formule générale MH avec des composés de formule générale III
H₂=CH-(CH₂)ₙ₋₂-(O)ₘ-[-D-B-]_{q}-Y, (III)
en présence d'au moins un métal du groupe du platine et/ou de ses composés, formule dans laquelle M, A, R, n, m, D, B, q, Y et p ont les significations énoncées dans les formules générales I et II mentionnées ci-dessus dans les revendications 1 à 3, à condition que l'atome d'hydrogène dans MH soit lié à un atome de silicium.

5. Procédé de préparation de composés de formule générale I selon l'une quelconque des revendications 1 à 3, qui consiste à faire réagir des composés de formule générale M-Hal dans laquelle Hal représente un atome de chlore, de brome ou d'iode, avec des composés de formule générale IV
T-(CH₂)ₙ- (O)ₘ-[-D-B-]_{q}-Y (IV)
dans laquelle T représente un atome de métal alcalin ou Mg-Hal, où Hal a les significations mentionnées ci-dessus, avec élimination d'un halogénure métallique T-Hal, où M, A, R, n, m, D, B, q, Y et p ont les significations énoncées dans les formules générales I et II mentionnées ci-dessus dans les revendications 1 à 3, à condition que, dans le cas où B et/ou Y réagissent avec des métaux alcalins ou du magnésium ou des composés de formule générale IV, B et/ou Y ne soient introduits qu'après qu'ait eu lieu la réaction de M-Hal avec les composés de formule générale IV.

6. Procédé de préparation des composés de formule générale I selon l'une quelconque des revendications 1 à 3, qui consiste à faire réagir des composés de formule générale V
M-(CH₂)ₙ-(O)ₘ-[-D-B-]ᵣ₋₁-D-G (V)
avec des composés de formule générale VI
Q-[-D-B-]ₛ-Y (VI)
formules dans lesquelles r et s sont chacun des nombres entiers de 0 à 3 dont le total est q, G et Q représentent chacun l'un des radicaux -OH, -OLi, -ONa, -OK, -O-alkyle (en C₁ à C₄), -COOH, -COBr, -COCl, -NH₂, -O-tos ou -MgHal, et M, A, R, n, m, D, B, q, Y et p ont les significations énoncées dans les formules générales I et II mentionnées ci-dessus dans les revendications 1 à 3 et Hal a la signification énoncée dans la revendication 5, pour former des groupes de liaison B et éliminer des composés W qui représentent l'eau, un alcanol en C₁-C₄, HHal, MgHal₂, LiHal, NaHal, KHal, Li-O-tos, Na-O-tos ou K-O-tos.

7. Compositions cristallines liquides qui comprennent au moins un composé selon l'une quelconque des revendications 1 à 3 ou un composé ayant été préparé par le procédé selon l'une quelconque des revendications 4 à 6 et qui présentent des phases nématiques, smectiques et/ou cholestériques.

8. Utilisation des composés selon l'une quelconque des revendications 1 à 3 ou des composés pouvant être préparés par le procédé selon l'une quelconque des revendications 4 à 6 et des compositions cristallines liquides selon la revendication 7, en optoélectronique, stockage des informations, production de signaux ou dans des procédés électrographiques.
